# EUROPEAN PATENT SPECIFICATION

(11) **EP 2 637 633 B1**
(45) Date of publication and mention of the grant of the patent: **11.07.2018**
(21) Application number: 10779424.0
(22) Date of filing: 12.11.2010
(51) Int. Cl.: A61K 8/27, A61K 8/34, A61K 8/37, A61K 8/49, A61K 8/365, A61Q 11/00

(54) **ORAL CARE PRODUCT AND METHODS OF USE AND MANUFACTURE THEREOF**
MUNDPFLEGEPRODUKT UND VERFAHREN ZU SEINER VERWENDUNG UND HERSTELLUNG
PRODUIT POUR SOINS BUCCAUX, ET SES PROCÉDÉS D'UTILISATION ET DE FABRICATION

(43) Date of publication of application: 18.09.2013
(73) Proprietor: Colgate-Palmolive Company, New York, NY 10022 (US)
(72) Inventor: LEWUS, Catherine, Denville New Jersey 07834 (US); SZEWCZYK, Gregory, Flemington New Jersey 08822 (US); MELLO, Sarita, North Brunswick New Jersey 08902 (US); SMITH-WEBSTER, Kimdra, Williamstown New Jersey 08094 (US); NESTA, Jason, Cedar Knolls New Jersey 07927 (US); DILLON, Rensl, Ewing New Jersey 08638 (US); ARVANITIDOU, Evangelia, S., Princeton New Jersey 08540 (US); CUIULE, Christine, Mount Laurel New Jersey 08904 (US)
(74) Representative: Wibbelmann, Jobst
(86) International application number: PCT/US2010/056518
(87) International publication number: WO 2012/064341

(56) References cited:
- EP-A2- 2 065 025
- WO-A2-2007/139950
- US-A- 4 289 755
- US-A- 5 817 295
- US-A1- 2009 035 229
- US-A1- 2010 286 218
- US-B1- 7 645 746
- DATABASE GNPD [Online] Mintel; October 2010 (2010-10), "Antiseptic Mouthwash with Mint Falvour", XP002659928, Database accession no. 1409771

## Description

### FIELD OF THE INVENTION

This invention relates to a mouthwash as defined in the claims as well as to its use in different methods as defined in the claims.

### BACKGROUND OF THE INVENTION

Because of their high water content, mouthwashes present particular challenges in preventing microbial contamination. While zinc salts are known to have antimicrobial activity-this is indeed their main function in the mouthwash - they are not effective against all microbial challenges, and it is therefore desirable to include preservatives in the formulation. Selection of the preservative is not trivial, however, as the zinc ions may react with formulation ingredients to form insoluble precipitates or chemically unavailable complexes, impairing the effectiveness of the zinc or the preservative or both. Additionally, some preservatives negatively affect the taste or aesthetics of the product. Finally, conventional agents such as ethanol and paraben preservatives may be undesirable for certain indication or in particular markets.

Accordingly, there is a need to identify improved preservative agents for use in mouthwashes comprising zinc salts. Zinc-containing mouthwashes are e.g., known from US 5,817,295 A and US 4,289,755 A.

### BRIEF SUMMARY OF THE INVENTION

It is now surprisingly discovered that mouthwashes comprising a soluble zinc salt and a preservative selected from selected from methylisothiazolinone, benzyl alcohol, glycerol monocaprylate, and combinations thereof, are stable and effective. The zinc salt may be, for example, any orally acceptable source of soluble zinc ions, e.g., zinc citrate.

The invention thus relates to mouthwashes and said mouthwashes for use in methods as defined in the claims.

The invention thus provides a mouthwash (a Composition of the Invention) according to claim 1.

Preferred features are defined in the dependent claims.

The Compositions of the Invention may comprise additional ingredients, e.g., selected from one or more of water, surfactants, solvents, vitamins, minerals, polymers, enzymes, humectants, thickeners, additional antimicrobial agents, additional preservatives, flavorings, colorings and/or combinations thereof. In particular embodiments, the invention may comprise an anti-calculus agent for example polyphosphate, e.g., pyrophosphate, tripolyphosphate, or hexametaphosphate, e.g., in alkali, e.g., sodium or potassium salt form, and/or may comprise a synthetic anionic polymeric polycarboxylate, such as 1:4 to 4:1 copolymers of maleic anhydride or acid with another polymerizable ethylenically unsaturated monomer, for example a co-polymer of methyl vinyl ether/maleic anhydride.

Glycerol monocaprylate is an octanoic acid monoester with glycerol, commercially available as Lexgard GMCY (Innolex Chemical). Methylisothiazolinone (MIT) and benzyl alcohol are readily available from a variety of sources.

The invention further encompasses a mouthwash according to the present invention for use in a method as defined in the claims.

The methods may comprise applying compositions effective upon application to the oral cavity, e.g., rinsing the oral cavity, optionally in conjunction with brushing.

### DETAILED DESCRIPTION OF THE INVENTION

The invention thus provides, in a first embodiment, a mouthwash (Composition 1.0) as defined in claim 1.

Particular embodiments are described in the following:
1.0.2. The foregoing composition wherein the zinc salt is dissolved zinc citrate.
1.0.3. The foregoing composition wherein the zinc salt is dissolved zinc citrate trihydrate.
1.0.4. Any of the foregoing compositions 1.0.2 and 1.0.4 wherein the zinc salt is present from 0.1 to 1 wt%.
1.0.6. The foregoing composition wherein the preservative is MIT.
1.0.7. Any of the foregoing compositions further comprising an anti-calculus agent for example polyphosphate, e.g., pyrophosphate, tripolyphosphate, or hexametaphosphate, e.g., in salt form, e.g., sodium or potassium salt form, e.g., in an amount of from 0.1 - 3%.
1.0.8. The foregoing composition wherein the anti-calculus agent is a pyrophosphate selected from tetrasodium pyrophosphate and tetrapotassium pyrophosphate and mixtures thereof.
1.0.9. The foregoing composition comprising 0.1 to 1% tetrasodium pyrophosphate and 1 - 2% tetrapotassium pyrophosphate, e.g. 0.25 - 0.75% tetrasodium pyrophosphate and 1.0 - 1.5% tetrapotassium pyrophosphate.
1.0.10. Any of the preceding compositions comprising at least one polymer selected from polyethylene glycols; synthetic anionic polymeric polycarboxylate, such as polyvinylmethyl ether maleic acid copolymers; polysaccharides (e.g., cellulose derivatives, for example carboxymethyl cellulose or polysaccharide gums, for example xanthan gum or carrageenan gum); and combinations thereof.
1.0.11. Any of the foregoing compositions comprising a synthetic anionic polymeric polycarboxylate, e.g., in an amount of 1 - 10%, e.g., 2.5 - 7.5%.
1.0.12. The foregoing composition wherein the synthetic anionic polymeric polycarboxylate is a 1:4 to 4:1 copolymer of maleic anhydride or acid with another polymerizable ethylenically unsaturated monomer, e.g. methyl vinyl ether/maleic anhydride having a molecular weight (M.W.) of 30,000 to 5,000,000 daltons, for example 1000kD - 3000kD.
1.0.13. The foregoing composition comprising a co-polymer of methyl vinyl ether/maleic anhydride having the general structure -[-CH₂-CH(OCH₃)-CH(COOH)-CH(COOH)-⁆-ₙ, viscosity at 25°C of 1-3 Pa·s (1-3 kCP), e.g., 1.7 Pa·s (1.7x10³ CP), and a nominal molecular weight of 1000 kD-3000 kD, e.g., 1.98x10⁶, for example in an amount by weight of 1-10%, e.g., 5%.
1.0.14. Any of the foregoing compositions which is ethanol-free.
1.0.15. Any of the foregoing compositions further comprising a basic amino acid in free or salt for, for example arginine, for example in an amount of 0.1 - 3%, e.g. abot 0.8%,
1.0.16. Any of the foregoing compositions further comprising a soluble calcium salt, e.g., selected from calcium glycerophosphate and salts of soluble carboxylic acids, and mixtures thereof, e.g., wherein the calcium salt is selected from calcium citrate, calcium malate, calcium lactate, calcium formate, calcium fumarate, calcium gluconate, calcium lactate gluconate, calcium aspartate, and calcium propionate, and mixtures thereof.
1.0.17. Any of the preceding compositions further comprising a fluoride source, e.g., a fluoride salt, for example sodium fluoride, or wherein the fluoride is covalently bound to another atom, e.g., a monofluorophosphate, for example sodium monofluorophosphate, a fluorosilicate, e.g., sodium fluorosilicate or ammonium fluorosilicate, or a fluorosulfate, e.g., hexafluorosulfate, amine fluoride and combinations thereof.
1.0.18. The preceding composition wherein the fluoride salt is present in an amount to provide 100 to 250 ppm available fluoride.
1.0.19. Any of the preceding compositions comprising sodium fluoride in an amount of 0.01-0.1%, e.g., 0.05%.
1.0.20. Any of the preceding compositions wherein the pH is between 7 and 8, e.g. 7.5.
1.0.21. Any of the preceding compositions further comprising an abrasive or particulate.
1.0.22. Any of the preceding compositions comprising a nonionic surfactant, e.g., in an amount of from 0.5 -5%, for example 1-2%, selected from polaxamers (e.g., polaxamer 407), polysorbates (e.g., polysorbate 20), polyoxyl hydrogenated castor oil (e.g., polyoxyl 40 hydrogenated castor oil), and mixtures thereof.
1.0.23. Any of the preceding compositions comprising at least one humectant.
1.0.24. Any of the preceding compositions comprising at least one humectant selected from glycerin, sorbitol, propylene glycol, and combinations thereof, e.g., in a total amount of 10-40%.
1.0.25. Any of the preceding compositions comprising polymer films.
1.0.26. Any of the preceding compositions comprising flavoring, fragrance and/or coloring.
1.0.27. Any of the preceding compositions comprising at least 50% water.
1.0.28. Any of the preceding compositions comprising an antibacterial agent selected from halogenated diphenyl ether (e.g. triclosan), herbal extracts and essential oils (e.g., rosemary extract, tea extract, magnolia extract, thymol, menthol, eucalyptol, geraniol, carvacrol, citral, hinokitol, catechol, methyl salicylate, epigallocatechin gallate, epigallocatechin, gallic acid, miswak extract, sea-buckthorn extract), bisguanide antiseptics (e.g., chlorhexidine, alexidine or octenidine), quaternary ammonium compounds (e.g., cetylpyridinium chloride (CPC), benzalkonium chloride, tetradecylpyridinium chloride (TPC), N-tetradecyl-4-ethylpyridinium chloride (TDEPC)), phenolic antiseptics, hexetidine, octenidine, sanguinarine, povidone iodine, delmopinol, salifluor, other metal ions (e.g., stannous salts, copper salts, iron salts), sanguinarine, propolis and oxygenating agents (e.g., hydrogen peroxide, buffered sodium peroxyborate or peroxycarbonate), phthalic acid and its salts, monoperthalic acid and its salts and esters, ascorbyl stearate, oleoyl sarcosine, alkyl sulfate, dioctyl sulfosuccinate, salicylanilide, domiphen bromide, delmopinol, octapinol and other piperidino derivatives, nicin preparations, chlorite salts; and mixtures of any of the foregoing.
1.0.29. Any of the preceding compositions comprising an antioxidant, e.g., selected from the group consisting of Co-enzyme Q10, PQQ, Vitamin C, Vitamin E, Vitamin A, BHT, anethole-dithiothione, and mixtures thereof.
1.0.30. Any of the preceding compositions comprising a whitening agent.
1.0.31. Any of the preceding compositions comprising a whitening agent selected from a whitening active selected from the group consisting of peroxides, metal chlorites, perborates, percarbonates, peroxyacids, hypochlorites, and combinations thereof.
1.0.32. Any of the preceding compositions further comprising hydrogen peroxide or a hydrogen peroxide source, e.g., urea peroxide or a peroxide salt or complex (e.g., such as peroxyphosphate, peroxycarbonate, perborate, peroxysilicate, or persulphate salts; for example calcium peroxyphosphate, sodium perborate, sodium carbonate peroxide, sodium peroxyphosphate, and potassium persulfate), or hydrogen peroxide polymer complexes such as hydrogen peroxide-polyvinyl pyrrolidone polymer complexes.
1.0.33. Any of the preceding compositions further comprising an agent that interferes with or prevents bacterial attachment, e.g., ELA or chitosan.
1.0.34. Any of the preceding compositions further comprising a physiologically acceptable potassium salt, e.g., potassium nitrate or potassium chloride, in an amount effective to reduce dentinal sensitivity.
1.0.35. Any of the preceding compositions comprising from 0.01% to 1% of a physiologically acceptable potassium salt, e.g., potassium nitrate and/or potassium chloride.
1.0.36. Any of the preceding compositions effective upon application to the oral cavity, e.g., by rinsing, optionally in conjunction with brushing, to (i) reduce or inhibit formation of dental caries, (ii) reduce, repair or inhibit pre-carious lesions of the enamel, e.g., as detected by quantitative light-induced fluorescence (QLF) or electrical caries measurement (ECM), (iii) reduce or inhibit demineralization and promote remineralization of the teeth, (iv) reduce hypersensitivity of the teeth, (v) reduce or inhibit gingivitis, (vi) promote healing of sores or cuts in the mouth, (vii) reduce levels of acid producing bacteria, (viii) to increase relative levels of arginolytic bacteria, (ix) inhibit microbial biofilm formation in the oral cavity, (x) raise and/or maintain plaque pH at levels of at least pH 5.5 following sugar challenge, (xi) reduce plaque accumulation, (xii) treat, relieve or reduce dry mouth, (xiii) reduce erosion, (xiv) prevents stains and/or whiten teeth, (xv) immunize the teeth against cariogenic bacteria; and/or (xvi) promote systemic health, including cardiovascular health, e.g., by reducing potential for systemic infection via the oral tissues.
1.0.37. A composition obtained or obtainable by combining the ingredients as set forth in any of the preceding compositions.

Levels of active ingredients will vary based on the nature of the delivery system and the particular active. The zinc salt is present at levels from 0.05 to 2 wt %, for example from 0.1 to 1 wt %. Fluoride may be present at levels of, e.g., 25 to 250 ppm, or up to 10x higher for a professional or prescription treatment product. Levels of additional antibacterial will vary similarly, depending on the agent used. For example, a triclosan mouthrinse may contain, e.g., 0.03 wt % triclosan.

The invention also relates to a mouthwash as defined in any of claims 1 to 3, for use in a method to
a. reduce or inhibit formation of dental caries,
b. reduce or inhibit gingivitis,
c. promote healing of sores or cuts in the mouth,
d. inhibit microbial biofilm formation in the oral cavity,
e. reduce plaque accumulation, and/or
f. enhance systemic health, including cardiovascular health.

The compositions of the invention are intended for topical use in the mouth and so salts for use in the present invention should be safe for such use, in the amounts and concentrations provided. Suitable salts include salts known in the art to be pharmaceutically acceptable salts are generally considered to be physiologically acceptable in the amounts and concentrations provided. Physiologically acceptable salts include those derived from pharmaceutically acceptable inorganic or organic acids or bases, for example acid addition salts formed by acids which form a physiological acceptable anion, e.g., hydrochloride or bromide salt, and base addition salts formed by bases which form a physiologically acceptable cation, for example those derived from alkali metals such as potassium and sodium or alkaline earth metals such as calcium and magnesium. Physiologically acceptable salts may be obtained using standard procedures known in the art, for example, by reacting a sufficiently basic compound such as an amine with a suitable acid affording a physiologically acceptable anion.

Fluoride Ion Source: The oral care compositions may further include one or more fluoride ion sources, e.g., soluble fluoride salts. A wide variety of fluoride ion-yielding materials can be employed as sources of soluble fluoride in the present compositions. Examples of suitable fluoride ion-yielding materials are found in U.S. Pat. No. 3,535,421, to Briner et al.; U.S. Pat. No. 4,885,155, to Parran, Jr. et al. and U.S. Pat. No. 3,678,154, to Widder et al. Representative fluoride ion sources include, but are not limited to, stannous fluoride, sodium fluoride, potassium fluoride, sodium monofluorophosphate, sodium fluorosilicate, ammonium fluorosilicate, amine fluoride, ammonium fluoride, and combinations thereof. In certain embodiments the fluoride ion source includes stannous fluoride, sodium fluoride, sodium monofluorophosphate as well as mixtures thereof. Where the formulation comprises calcium salts, the fluoride salts are preferably salts wherein the fluoride is covalently bound to another atom, e.g., as in sodium monofluorophosphate, rather than merely ionically bound, e.g., as in sodium fluoride.

### Surfactants

The invention may in some embodiments contain anionic surfactants, for example, water-soluble salts of higher fatty acid monoglyceride monosulfates, such as the sodium salt of the monosulfated monoglyceride of hydrogenated coconut oil fatty acids such as sodium N-methyl N-cocoyl taurate, sodium cocomo-glyceride sulfate; higher alkyl sulfates, such as sodium lauryl sulfate; higher alkyl-ether sulfates, e.g., of formula CH₃(CH₂)ₘCH₂(OCH₂CH₂)ₙOSO₃X, wherein m is 6-16, e.g., 10, n is 1-6, e.g., 2, 3 or 4, and X is Na or K, for example sodium laureth-2 sulfate (CH₃(CH₂)₁₀CH₂(OCH₂CH₂)₂OSO₃Na); higher alkyl aryl sulfonates such as sodium dodecyl benzene sulfonate (sodium lauryl benzene sulfonate); higher alkyl sulfoacetates, such as sodium lauryl sulfoacetate (dodecyl sodium sulfoacetate), higher fatty acid esters of 1,2 dihydroxy propane sulfonate, sulfocolaurate (N-2-ethyl laurate potassium sulfoacetamide) and sodium lauryl sarcosinate. By "higher alkyl" is meant, e.g., C₆₋₃₀ alkyl. In particular embodiments, the anionic surfactant (where present) is selected from sodium lauryl sulfate and sodium ether lauryl sulfate. When present, the anionic surfactant is present in an amount which is effective, e.g., > 0.001% by weight of the formulation, but not at a concentration which would be irritating to the oral tissue, e.g., < 1%, and optimal concentrations depend on the particular formulation and the particular surfactant. In one embodiment, the anionic surfactant is present at from 0.03% to 0.5% by weight, e.g., 0.15%.

In another embodiment, cationic surfactants useful in the present invention can be broadly defined as derivatives of aliphatic quaternary ammonium compounds having one long alkyl chain containing 8 to 18 carbon atoms such as lauryl trimethylammonium chloride, cetyl pyridinium chloride, cetyl trimethylammonium bromide, di-isobutylphenoxyethyldimethylbenzylammonium chloride, coconut alkyltrimethylammonium nitrite, cetyl pyridinium fluoride, and mixtures thereof. Illustrative cationic surfactants are the quaternary ammonium fluorides described in U.S. Pat. No. 3,535,421, to Briner et al. Certain cationic surfactants can also act as germicides in the compositions.

Illustrative nonionic surfactants that can be used in the compositions of the invention can be broadly defined as compounds produced by the condensation of alkylene oxide groups (hydrophilic in nature) with an organic hydrophobic compound which may be aliphatic or alkylaromatic in nature. Examples of suitable nonionic surfactants include, but are not limited to, the Pluronics, polyethylene oxide condensates of alkyl phenols, products derived from the condensation of ethylene oxide with the reaction product of propylene oxide and ethylene diamine, ethylene oxide condensates of aliphatic alcohols, long chain tertiary amine oxides, long chain tertiary phosphine oxides, long chain dialkyl sulfoxides and mixtures of such materials. In a particular embodiment, the composition of the invention comprises a nonionic surfactant selected from polaxamers (e.g., polaxamer 407), polysorbates (e.g., polysorbate 20), polyoxyl hydrogenated castor oils (e.g., polyoxyl 40 hydrogenated castor oil), and mixtures thereof.

In certain embodiments, zwitterionic synthetic surfactants useful in the present invention can be broadly described as derivatives of aliphatic quaternary ammonium, phosphomium, and sulfonium compounds, in which the aliphatic radicals can be straight chain or branched, and wherein one of the aliphatic substituents contains 8 to 18 carbon atoms and one contains an anionic water-solubilizing group, e.g., carboxy, sulfonate, sulfate, phosphate or phosphonate.

Illustrative examples of the surfactants suited for inclusion into the composition include, but are not limited to, sodium alkyl sulfate, sodium lauroyl sarcosinate, cocoamidopropyl betaine and polysorbate 20, and combinations thereof.

The surfactant or mixtures of compatible surfactants can be present in the compositions of the present invention in 0.1% to 5%, in another embodiment 0.3% to 3% and in another embodiment 0.5% to 2% by weight of the total composition.

### Flavoring Agents

The oral care compositions of the invention may also include a flavoring agent. Flavoring agents which are used in the practice of the present invention include, but are not limited to, essential oils and various flavoring aldehydes, esters, alcohols, and similar materials, as well as sweeteners such as sodium saccharin. Examples of the essential oils include oils of spearmint, peppermint, wintergreen, sassafras, clove, sage, eucalyptus, marjoram, cinnamon, lemon, lime, grapefruit, and orange. Also useful are such chemicals as menthol, carvone, and anethole. Certain embodiments employ the oils of peppermint and spearmint.

The flavoring agent is incorporated in the oral composition at a concentration of 0.01 to 1% by weight.

### Chelating and anti-plaque agents

The oral care compositions of the invention also may optionally include one or more chelating agents able to complex calcium found in the cell walls of the bacteria. Binding of this calcium weakens the bacterial cell wall and augments bacterial lysis.

One group of agents suitable for use as chelating or anti-plaque agents in the present invention are the soluble pyrophosphates. The pyrophosphate salts used in the present compositions can be any of the alkali metal pyrophosphate salts. In certain embodiments, salts include tetra alkali metal pyrophosphate, dialkali metal diacid pyrophosphate, trialkali metal monoacid pyrophosphate and mixtures thereof, wherein the alkali metals are sodium or potassium. The salts are useful in both their hydrated and unhydrated forms. An effective amount of pyrophosphate salt useful in the present composition is generally enough to provide at least 0.5 wt. % pyrophosphate ions, 0.9 - 3 wt. %.

These compounds also contribute to preservation of the compositions by lowering water activity.

### Polymers

The oral care compositions of the invention also optionally include one or more polymers, such as polyethylene glycols, polyvinylmethyl ether maleic acid copolymers, polysaccharides (e.g., cellulose derivatives, for example carboxymethyl cellulose, or polysaccharide gums, for example xanthan gum, gellan gum or carrageenan gum). Acidic polymers, for example polyacrylate gels, may be provided in the form of their free acids or partially or fully neutralized water soluble alkali metal (e.g., potassium and sodium) or ammonium salts.

In a particular embodiment, the compositions of the invention include synthetic anionic polymeric polycarboxylates, such as 1:4 to 4:1 copolymers of maleic anhydride or acid with another polymerizable ethylenically unsaturated monomer, preferably methyl vinyl ether/maleic anhydride having a molecular weight (M.W.) of 30,000 to 3,000,000, most preferably 30,000 to 800,000. These copolymers are available for example as Gantrez. e.g., AN 139 (M.W. 500,000), AN 119 (M.W. 250,000) and S-97 Pharmaceutical Grade (M.W. 700,000 to 1,500,000) available from ISP Technologies, Inc., Bound Brook, N.J. 08805. The enhancing agents when present are present in amounts of 1 to 10% by weight.

Other operative polymers include those such as the 1:1 copolymers of maleic anhydride with ethyl acrylate, hydroxyethyl methacrylate, N-vinyl-2-pyrollidone, or ethylene, the latter being available for example as Monsanto EMA No. 1103, M.W. 10,000 and EMA Grade 61, and 1:1 copolymers of acrylic acid with methyl or hydroxyethyl methacrylate, methyl or ethyl acrylate, isobutyl vinyl ether or N-vinyl-2-pyrrolidone.

Suitable generally, are polymerized olefinically or ethylenically unsaturated carboxylic acids containing an activated carbon-to-carbon olefinic double bond and at least one carboxyl group, that is, an acid containing an olefinic double bond which readily functions in polymerization because of its presence in the monomer molecule either in the alpha-beta position with respect to a carboxyl group or as part of a terminal methylene grouping. Illustrative of such acids are acrylic, methacrylic, ethacrylic, alpha-chloroacrylic, crotonic, beta-acryloxy propionic, sorbic, alpha-chlorsorbic, cinnamic, beta-styrylacrylic, muconic, itaconic, citraconic, mesaconic, glutaconic, aconitic, alpha-phenylacrylic, 2-benzyl acrylic, 2-cyclohexylacrylic, angelic, umbellic, fumaric, maleic acids and anhydrides. Other different olefinic monomers copolymerizable with such carboxylic monomers include vinylacetate, vinyl chloride, dimethyl maleate and the like. Copolymers contain sufficient carboxylic salt groups for water-solubility.

A further class of polymeric agents includes a composition containing homopolymers of substituted acrylamides and/or homopolymers of unsaturated sulfonic acids and salts thereof, in particular where polymers are based on unsaturated sulfonic acids selected from acrylamidoalykane sulfonic acids such as 2-acrylamide 2 methylpropane sulfonic acid having a molecular weight of 1,000 to 2,000,000, described in U.S. Pat. No. 4,842,847, Jun. 27, 1989 to Zahid.
Another useful class of polymeric agents includes polyamino acids, particularly those containing proportions of anionic surface-active amino acids such as aspartic acid, glutamic acid and phosphoserine, as disclosed in U.S. Pat. No. 4,866,161 Sikes et al.

In preparing oral care compositions, it is sometimes necessary to add some thickening material to provide a desirable consistency or to stabilize or enhance the performance of the formulation. In certain embodiments, the thickening agents are carboxyvinyl polymers, carrageenan, hydroxyethyl cellulose and water soluble salts of cellulose ethers such as sodium carboxymethyl cellulose and sodium carboxymethyl hydroxyethyl cellulose. Natural gums such as karaya, gum arabic, and gum tragacanth can also be incorporated. Colloidal magnesium aluminum silicate or finely divided silica can be used as component of the thickening composition to further improve the composition's texture. In certain embodiments, thickening agents in an amount of 0.5% to 5% by weight of the total composition are used.

### Enzymes

The oral care compositions of the invention may also optionally include one or more enzymes. Useful enzymes include any of the available proteases, glucanohydrolases, endoglycosidases, amylases, mutanases, lipases and mucinases or compatible mixtures thereof. In certain embodiments, the enzyme is a protease, dextranase, endoglycosidase and mutanase. In another embodiment, the enzyme is papain, endoglycosidase or a mixture of dextranase and mutanase. Additional enzymes suitable for use in the present invention are disclosed in U.S. Pat. No. 5,000,939 to Dring et al., U.S. Pat. No. 4,992,420; U.S. Pat. No. 4,355,022; U.S. Pat. No. 4,154,815; U.S. Pat. No. 4,058,595; U.S. Pat. No. 3,991,177; and U.S. Pat. No. 3,696,191. An enzyme of a mixture of several compatible enzymes in the current invention constitutes 0.002% to 2.0% in one embodiment or 0.05% to 1.5% in another embodiment or in yet another embodiment 0.1% to 0.5%.

### Water

Water is present in the oral compositions of the invention. Water, employed in the preparation of commercial oral compositions should be deionized and free of organic impurities. Water commonly makes up the balance of the compositions and includes 10% to 90%, e.g., 40% to 70% by weight of the oral compositions. This amount of water includes the free water which is added plus that amount which is introduced with other materials such as with sorbitol or any components of the invention.

### Humectants

Within certain embodiments of the oral compositions, it is also desirable to incorporate a humectant to reduce evaporation and also contribute towards preservation by lowering water activity. Certain humectants can also impart desirable sweetness or flavor to compositions. The humectant, on a pure humectant basis, generally includes 15% to 70% in one embodiment or 30% to 65% in another embodiment by weight of the composition.

Suitable humectants include edible polyhydric alcohols such as glycerine, sorbitol, xylitol, propylene glycol as well as other polyols and mixtures of these humectants. Mixtures of glycerine and sorbitol may be used in certain embodiments as the humectant component of the compositions herein.

The present invention in its method aspect involves applying to the oral cavity a safe and effective amount of the compositions described herein.

The compositions and methods according to the invention are useful to a method to protect the teeth by facilitating repair and remineralization, in particular to reduce or inhibit formation of dental caries, reduce or inhibit demineralization and promote remineralization of the teeth, reduce hypersensitivity of the teeth, and reduce, repair or inhibit early enamel lesions, e.g., as detected by quantitative light-induced fluorescence (QLF) or electronic caries monitor (ECM).

Test methods for the desensitizing properties of the compositions described herein, uses the method described in U.S. Pat. No. 5,589,159. This method measures the hydraulic conductance of materials, providing an objective reduction in fluid flow that correlates with reduction in fluid flow in dentinal tubules. In this method, intact human molars free from caries and restorations are sectioned perpendicularly to the long axis of the tooth with a metallurgical saw to form thin sections, or discs, from about 0.4 to about 0.8 mm thick. Sections containing dentin and free of enamel were selected for testing and then etched with citric acid solution to remove the smear layer. Each disc was mounted into a split chambered device described in J. Dent. Research, 57:187 (1978) which is a special leak-proof chamber connected to a pressurized fluid reservoir containing a tissue culture fluid. By using a mixture of pressurized nitrogen and carbon dioxide gas, the fluid can be made at physiological pH. To further ensure accuracy, the discs were wetted with artificial saliva (phosphate buffer saline , PBS) to approximate intra-oral conditions. The apparatus includes a glass capillary tube attached to a flow sensor (FLODEC, DeMarco Engineering SA, Geneva). An air bubble is injected into the glass capillary tube. By measuring the displacement of the bubble as a function of time, fluid flow through the dentin disc can be measured. Fluid flow is equivalent to the dentin permeability.

Enhancing oral health also provides benefits in systemic health, as the oral tissues can be gateways for systemic infections. Good oral health is associated with systemic health, including cardiovascular health. The compositions and methods of the invention are thus useful to enhance systemic health, including cardiovascular health.

As used throughout, ranges are used as shorthand for describing each and every value that is within the range. Any value within the range can be selected as the terminus of the range. In the event of a conflict in a definition in the present disclosure and that of a cited reference, the present disclosure controls. It is understood that when formulations are described, they may be described in terms of their ingredients, as is common in the art, notwithstanding that these ingredients may react with one another in the actual formulation as it is made, stored and used, and such products are intended to be covered by the formulations described.

The following examples further describe and demonstrate illustrative embodiments within the scope of the present invention. The examples are given solely for illustration and are not to be construed as limitations of this invention as many variations are possible without departing from the scope thereof. Various modifications of the invention in addition to those shown and described herein should be apparent to those skilled in the art and are intended to fall within the appended claims.

### EXAMPLE 1 - Mouthwashes

Formulations of the invention are prepared with the following ingredients:

| RAW MATERIAL | WEIGHT % |
|---|---|
| Sorbitol (70% solution) | 10 |
| Glycerin | 10 |
| Propylene glycol | 7 |
| Zinc citrate trihydrate | 0.28 |
| Gantrez S-97 (13% Solution) | 1.92 |
| Pyrophosphate salts | 1.7 |
| Sodium fluoride | 0.05 |
| Sodium saccharin | 0.025 |
| Surfactant | 0.5 |
| Flavor | 0.145 |
| Dye | 0.0003 |
| Methylisothiazolinone (MIT) | 0.01 |
| Water | Balance |
| | |
| TOTAL | 100 |
| Water activity | <95% |
| pH | 7.5 |

To optimize the preservative system, the methylisothiazolinone (MIT) is substituted with different preservatives in the above formulation, and the characteristics of the formulation tested for antimicrobial efficacy, flavor impact, and aesthetics impact.

The Antimicrobial Preservation Effectiveness Test is used to determine the antimicrobial preservation effectiveness of water-based product formulations by means of a double challenge test. Products are developed to withstand microbial challenges introduced by normal consumer use. The test is run on an aged sample (13 weeks, 40°C). The test uses two pools of microorganisms: bacteria/ yeast and mold. The product is challenged at a 1 % level at day 0 and at day 7. Reduction of the inoculum is monitored over a 28 day period. The following are the acceptance criteria for mouth wash formulas.
- Bacteria and Yeast must show a 99.9% reduction (3 logs) of the bacterial inoculum as determined by plate count on day 7 following each inoculation. No increase after day 7 of the second inoculation and for the remainder of the test within normal variation of the data.
- Mold must show a 90.0% reduction (1 log) of the mold inoculum as determined by the plate count on day 14 following the second inoculation (day 21). No increase from day 14 to day 21 of the second inoculation of the test within normal variation of the data.

Flavor is evaluated via an organoleptic evaluation by trained flavorists.

Aesthetics are evaluated by a visual comparison to a control sample having the same types and levels of colorant and flavoring agents.

Results of the comparative testing are as follows, where a "√" indicates criteria were met, and an "X" indicates criteria were not met.

| Preservative | Na benzoate | K sorbate | MIT | Benzyl alcohol | Phenoxyethanol | Polyamidopropyl Bisguanide | Lexgard/gmcy |
|---|---|---|---|---|---|---|---|
| Micro robustness | X | X | √ | √ | X | X | √ |
| In vitro eff. | √ | √ | √ | √ | √ | √ | √ |
| Flavor impact | √ | √ | X | slight | X | X | X |
| Aesthetics impact | √ | √ | √ | √ | √ | X | X |

| | | | | | | | |
|---|---|---|---|---|---|---|---|
| These results show that the different preservative systems behave unpredictably in the mouthwash formulations. The only preservatives which provided acceptable microbial control are benzyl alcohol, methylisothiazolinone (MIT), and glycerol monocaprylate (Lexgard GMCY®), separately or in combination. The adverse impact of methylisothiazolinone (MIT) on flavor is reduced by reducing the amount to 0.001%, while retaining antimicrobial efficacy. | | | | | | | |

## Claims

1. A mouthwash comprising an aqueous solution of
a) an effective amount of an orally acceptable soluble zinc salt, wherein the zinc salt is
present in an amount corresponding to 0.05 to 2 wt. %; and
b) an effective amount of a preservative selected from methylisothiazolinone (MIT),
benzyl alcohol, glycerol monocaprylate, and combinations thereof, wherein the preservatives are present, separately or in combination, in amounts as follows:
i) MIT: 0.0005 - 0.1 wt. %
ii) benzyl alcohol: 0.05 - 0.25 wt.%
iii) glycerol monocaprylate: 0.01 % - 0.25 wt.%;
wherein
A) the zinc salt is zinc citrate; or
B) the mouthwash further comprises a pyrophosphate; or
C) the mouthwash further comprises a synthetic anionic polymeric polycarboxylate; or
D) the mouthwash further comprises a fluoride ion source.

2. A mouthwash according to claim 1 which is ethanol-free.

3. A mouthwash according to any of the preceding claims further comprising one or more of
humectants, flavorings, and surfactants.

4. A mouthwash according to any of claims 1 to 3, for use in a method to
a. reduce or inhibit formation of dental caries,
b. reduce or inhibit gingivitis,
c. promote healing of sores or cuts in the mouth,
d. inhibit microbial biofilm formation in the oral cavity,
e. reduce plaque accumulation, and/or
f. enhance systemic health, including cardiovascular health.

## Patentansprüche

1. Mundwasser, umfassend eine wässrige Lösung von
a) einer wirksamen Menge eines oral verträglichen löslichen Zinksalzes, wobei das Zinksalz in einer Menge vorliegt, die 0,05 bis 2 Gew.-% entspricht; und
b) einer wirksamen Menge eines Konservierungsmittels, das aus Methylisothiazolinon (MIT), Benzylalkohol, Glycerinmonocaprylat und Kombinationen davon ausgewählt ist, wobei die Konservierungsmittel separat oder in Kombination in Mengen vorliegen wie folgt:
i) MIT: 0,0005 - 0,1 Gew.-%
ii) Benzylalkohol: 0,05 - 0,25 Gew.-%
iii) Glycerinmonocaprylat: 0,01% - 0,25 Gew.-%;
wobei
A) das Zinksalz Zinkcitrat ist; oder
B) das Mundwasser weiterhin ein Pyrophosphat umfasst; oder
C) das Mundwasser weiterhin ein synthetisches anionisches polymeres Polycarboxylat umfasst; oder
D) das Mundwasser weiterhin eine Fluoridionenquelle umfasst.

2. Mundwasser nach Anspruch 1, das ethanolfrei ist.

3. Mundwasser nach einem beliebigen der voranstehenden Ansprüche, das weiterhin ein oder mehrere Feuchthaltemittel, Aromastoffe und oberflächenaktive Mittel umfasst.

4. Mundwasser nach einem beliebigen der Ansprüche 1 bis 3, zur Verwendung in einem Verfahren, um
a. die Bildung von Zahnkaries zu verringern oder zu inhibieren,
b. Gingivitis zu verringern oder zu inhibieren,
c. das Heilen von Wunden oder Schnittwunden im Mund zu fördern,
d. mikrobielle Biofilmbildung in der Mundhöhle zu inhibieren,
e. Plaque-Ansammlung zu verringern, und/oder
f. systemische Gesundheit, einschließlich Herz-Kreislauf-Gesundheit, zu erhöhen.

## Revendications

1. Bain de bouche comprenant une solution aqueuse de
a) une quantité efficace d'un sel de zinc soluble acceptable par voie orale, dans lequel le sel de zinc est présent en une quantité correspondant à 0,05 à 2 % en poids ; et
b) une quantité efficace d'un conservateur choisi parmi la méthylisothiazolinone (MIT), l'alcool benzylique, le monocaprylate de glycérol, et les combinaisons de ceux-ci, dans lequel les conservateurs sont présents, séparément ou en combinaison, en des quantités comme suit :
i) MIT : 0,0005 à 0,1 % en poids
ii) alcool benzylique : 0,05 à 0,25 % en poids
iii) monocaprylate de glycérol : 0,01 % à 0,25 % en poids ;
dans lequel
A) le sel de zinc est le citrate de zinc ; ou
B) le bain de bouche comprend en outre un pyrophosphate ; ou
C) le bain de bouche comprend en outre un polycarboxylate polymère anionique synthétique ; ou
D) le bain de bouche comprend en outre une source d'ions fluorure.

2. Bain de bouche selon la revendication 1, qui est exempt d'éthanol.

3. Bain de bouche selon l'une quelconque des revendications précédentes, comprenant en outre un ou plusieurs éléments parmi les humectants, les arômes et les tensioactifs.

4. Bain de bouche selon l'une quelconque des revendications 1 à 3, pour une utilisation dans une méthode pour
a. réduire ou inhiber la formation de caries dentaires,
b. réduire ou inhiber la gingivite,
c. favoriser la guérison de plaies ou de coupures dans la bouche,
d. inhiber la formation de biofilm microbien dans la cavité buccale,
e. réduire l'accumulation de la plaque, et/ou
F. améliorer la santé systémique, y compris la santé cardiovasculaire.
